# EUROPÄISCHE PATENTSCHRIFT

(11) **EP 0 614 665 B1**
(45) Veröffentlichungstag und Bekanntmachung des Hinweises auf die Patenterteilung: **16.06.1999**
(21) Anmeldenummer: 94102995.1
(22) Anmeldetag: 28.02.1994
(51) Int. Cl.: A61K 35/78, A23L 1/30

(54) **Verwendung von Biertreberextrakt zur Herstellung von oral zu verabreichenden Zubereitungen**
Use of beer draff extract for the preparation of oral compositions
Utilisation d'un extrait de drêche de bière pour la préparation de compositions orales

(30) Priorität: 05.03.1993 DE 4306931
(43) Veröffentlichungstag der Anmeldung: 14.09.1994
(73) Patentinhaber: Pentapharm AG, 4002 Basel (CH)
(72) Erfinder: Driller, Hansjürgen, Dr., D-40789 Monheim-Baumberg (DE); Tronnier, Hagen, Prof. Dr., D-58313 Herdecke (DE)
(74) Vertreter: Braun, André, jr.

(56) Entgegenhaltungen:
- EP-A- 0 287 003
- DE-A- 2 403 203
- COSMETICS & TOILETRIES Bd. 105 , November 1990 Seiten 59 - 62 L. MOTITSCHKE ET AL. 'SPENT GRAIN WAX'

## Beschreibung

Die Europäische Patentschrift 0 287 003 beschreibt die Verwendung von Biertreberextrakt zur Herstellung von medizinischen und kosmetischen Zubereitungen zur äußerlichen Anwendung. Als Anwendungsformen werden Lösungen, Suspensionen, Emulsionen, Pasten, Salben, Gelees, Cremes, Lotionen, Puder, Seife, tensidhaltige Reinigungspräparate, Öle und Sprays genannt.

Es wurde nun gefunden, daß oral zu verabreichende Zubereitungen von Biertreberextrakt bei Hautkrankheiten, die mit Juckreiz und/oder entzündlichen Erscheinungen einhergehen, zu einer Normalisierung des Fettsäurestoffwechsels führen und eine Verbesserung des Hautzustandes bewirken, die in einer Verbesserung des transepidermalen Wasserverlusts und der Hydratation der Haut zu erkennen ist sowie eine Abnahme der großen Hautfalten mit gleichzeitiger Zunahme der normalen Hautfältelung ergibt.

Die Erfindung betrifft die Verwendung von Biertreberextrakt zur Herstellung von oral zu verabreichenden medizinischen Zubereitungen zur Behandlung von bestimmten, insbesondere mit Juckreiz und/oder entzündlichen Erscheinungen einhergehenden Hautkrankheiten sowie zur Herstellung von oral zu verabreichenden diätetischen "Lebensmitteln" zur Unterstützung von besonderen Ernährungszwecken.

Diätetische Lebensmittel sind Lebensmittel, die bestimmt sind, einem besonderen Ernährungszweck dadurch zu dienen, daß sie die Zufuhr bestimmter Nährstoffe oder anderer ernährungsphysiologisch wirkender Stoffe steigern oder verringern oder die Zufuhr solcher Stoffe in einem bestimmten Mischungsverhältnis oder in bestimmter Beschaffenheit bewirken. Diätetische Lebensmittel müssen sich von anderen Lebensmitteln vergleichbarer Art durch ihre Zusammensetzung oder ihre Eigenschaften maßgeblich unterscheiden.

Diätetische Lebensmittel dienen einem besonderen Ernährungszweck, wenn sie dazu beitragen, besonderen Ernährungserfordernissen aufgrund von Umständen wie Krankheit, Mangelerscheinung, Funktionsanomalie und Überempfindlichkeit gegen einzelne Lebensmittel oder deren Bestandteile während der Schwangerschaft und Stillzeit sowie beim Säugling und Kleinkind zu entsprechen.

Der Biertreberextrakt kann mittels beliebigem Getreide (z. B. Gerste, Weizen, Hafer, Reis, Mais) hergestellt werden; vorzuziehen sind jedoch bestimmte Gerstensorten wie Hordeum distichum, Hordeum tetrastichum oder Hordeum hexastichum, insbesondere Hordeum distichum. Der Biertreber läßt sich naß oder trocken, vorzugsweise trocken extrahieren. Als Extraktionsmittel eignen sich Extraktionsmittel wie Alkohole, Ether, Ketone, Wasser, gegebenenfalls mit Zusatz von sauren oder basischen Stoffen. Darüber hinaus eignen sich auch andere Lösungsmittel wie Kohlenwasserstoffe (z. B. Petrolether, Hexan, Toluol) niedere halogenierte aliphatische Kohlenwasserstoffe (z. B. Dichlormethan, Trichlorfluormethan), Ester (z. B. Ameisensäureethylester, Essigsäuremethylester, Essigsäureethylester) und vorzugsweise sowohl CO₂ als auch N₂O im flüssigen oder überkritischen Bereich; besonders bevorzugtes Lösungsmittel ist CO₂ im überkritischen Bereich.
Die Extraktion mit den herkömmlichen (unter Normalbedingungen flüssigen) Lösungsmitteln erfolgt auf an sich übliche Weise, z. B. in der französischen Patentschrift FR 2 379 282 beschrieben. Die Extraktion mittels flüssigem oder überkritischem CO₂ erfolgt ebenfalls auf übliche Weise, z. B. in "Carbon dioxide extracted ingredients for fragrances" (Informationsschrift Pauls Flavours & Fragrances 0-5M385P, North Albert Road, Reigate, Surrey RH2 9ER, England) oder "Hochdruck-Extraktion" (Informationsschrift Hi 5 19200085 - HT80-5d + e + f Uhde GmbH, Buschmühlenstr. 20, 5800 Hagen 1) beschrieben; bevorzugte Parameter zur Biertreberextraktion mit überkritischem CO₂ sind Drucke von 75 bis 600 bar, insbesondere von 150 bis 350 bar, und Temperaturen von 31 bis 120°C, vorzugsweise von 40 bis 90°C; bevorzugte Parameter zur Biertreberextraktion mit flüssigem CO₂ sind Drucke von 50 bis 250 bar, insbesondere 100 bis 150 bar, und Temperaturen von -10 bis 31°C, insbesondere von 10 bis 20°C.

Die Extraktion mit flüssigem oder überkritischem N₂O wird praktisch in der gleichen Weise und unter den gleichen Bedingungen durchgeführt, wobei lediglich die bevorzugte Temperaturgrenze für das Arbeiten mit dem flüssigen und dem überkritischen N₂O geringfügig höher liegt - nämlich bei 36,5°C - als bei der Extraktion mit CO₂ (etwa 31°C). Die Extraktion erfolgt insbesondere wie in der Europäischen Patentschrift 0 287 003 beschrieben.
Die mittels herkömmlicher flüssiger Lösungsmittel gewonnenen Extrakte sind zu konzentrieren, z.B. durch Eindampfen. Die mit flüssigem oder überkritischem CO₂ bzw. N₂O gewonnenen Extrakte sind unter Normalbedingungen naturgemäß fast völlig frei von Extraktionsmittel. In der Zusammensetzung unterscheiden sich die einzelnen Extrakte laut dünnschichtchromatographischer Analyse kaum voneinander. Während die mittels herkömmlicher flüssiger Lösungsmittel gewonnenen Extrakte jedoch dunkel gefärbt sind und teilweise einen stechenden Geruch aufweisen, haben die CO₂- und N₂O-Extrakte eine goldgelbe Farbe und einen angenehmen, getreideartigen Geruch. Der Tropfpunkt der CO₂-Extrakte liegt im Bereich von 35 bis 39°C.

Für die oral zu verabreichende Zubereitung wird der Treberextrakt mit den dafür geeigneten Zusatzstoffen wie Trägerstoffen, Stabilisatoren oder inerten Verdünnungsmitteln sowie weiteren Hilfsstoffen vermischt und durch die üblichen Methoden in die geeigneten Darreichungsformen gebracht wie Tabletten, Dragees, Steckkapseln, wäßrige, alkoholische oder ölige Lösungen. Als inerte Träger werden z. B. Gummi arabicum, Magnesia, Magnesiumcarbonat, Kaliumphosphat, Milchzucker, Glucose oder Stärke, insbesondere Maisstärke, verwendet. Dabei kann die Zubereitung sowohl ein Trocken- als auch ein Feuchtgranulat sein. Als ölige Trägerstoffe oder als Lösemittel kommen beispielsweise pflanzliche oder tierische Öle in Betracht, wie Sonnenblumenöl oder Lebertran.

Welche Hilfsstoffe für die oral zu verabreichende Zubereitung geeignet sind, ist dem Fachmann aufgrund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Der obengenannte Treberextrakt kann beispielsweise auf bekannte Weise verkapselt werden. Geeignete Kapseln sind z. B. Hartgelatinekapseln, Weichgelatinekapseln oder Mikrokapseln (Seifen-Öle-Fette-Wachse, 1990, 116. Jahrgang, Nr. 5, Seiten 161 bis 168).

Die Konzentration des Biertreberextraktes in den medizinischen Zubereitungen beträgt 50 bis 100 Gew.-%, vorzugsweise 85 bis 95 Gew.-%.

Die Erfindung betrifft weiterhin die Verwendung von Biertreberextrakten zur Herstellung von diätetischen Lebensmitteln. Da es für ein diätetisches Lebensmittel besonders wichtig ist, einen angenehmen Geruch und eine ansprechende Färbung aufzuweisen, ist hier der CO₂- und der N₂O-Biertreberextrakt besonders wertvoll. Er kann, aufgrund seiner nur geringen Färbung und seines angenehmen Eigengeruchs, in höheren Konzentrationen als der herkömmliche Extrakt eingesetzt werden, ohne belästigend zu wirken.

Die Anwendungsformen der oral zu verabreichenden Zubereitungen sind im Prinzip die gleichen wie für die diätetischen Lebensmittel und umfassen weiterhin auch typisch diätetische Anwendungsformen wie Breie, Fertiggetränke oder Fertigpackungen für Säuglinge, Kleinkinder oder Erwachsene. Der Anteil an Biertreberextrakt beträgt vorzugsweise 30 bis 90 Gew.-%, insbesondere 60 bis 80 Gew.-%.

Die medizinischen oral zu verabreichenden Zubereitungen eignen sich als Mittel gegen Hautkrankheiten, vorzugsweise gegen Hautkrankheiten, die mit Juckreiz und/oder entzündlichen Erscheinungen einhergehen, insbesondere gegen Dermatitis atopica, Ichthyosis, Exsikkationsekzematoid, Pruritus, Sebostase und Dermatomykosen und Hautreizungen infolge von Quallenverbrennungen.

### Therapeutische Wirksamkeit im Vergleichsversuch

Patienten, die an Dermatitis atopica erkrankt sind, wurden in einer Gruppe aus 4 Patienten über 6 Monate mit den erfindungsgemäßen Biertreberextraktzubereitungen in der Form von Gelatinekapseln (Treber-Kapseln) und in einer Vergleichsgruppe aus 4 Patienten mit Nachtkerzenöl-Kapseln (Epogam ^{(R)}, Beiersdorf AG, Hamburg) behandelt. Die Herstellung der Treber-Kapseln erfolgte nach bekannten Methoden (A. Cuine et al., Pharm. Ind. 40 (1978) Seite 654 ff.). Der Nachtkerzenöl(NKO)-Gruppe wurden 8 bis 12 Kapseln mit jeweils 40 mg Z,Z,Z-9,12,15-Octadecantriensäure pro Kapsel täglich oral verabreicht, die Treber-Gruppe erhielt 6 bis 8 Treber-Kapseln mit etwa 44 mg Z,Z,Z-9,12,15-Octadecantriensäure pro Kapsel täglich verabreicht.

### A Bestimmung des transepidermalen Wasserverlustes

Diese Messungen erfolgten mit dem Evaporimeter an den unbehandelten Kontrollfeldern beider Unterarme sowie im Bereich der Hautveränderungen der Unterarmbeugen. Die Ergebnisse vor und während der 6-monatigen Behandlung mit NKO- oder Treber-Kapseln zeigt die folgende Tabelle 1:

**Tabelle 1**

| Transepidermaler Wasserverlust (g/hm²) | | | | |
|---|---|---|---|---|
| NKO-Kapseln | vorher | nach 2 Monaten | nach 4 Mon. | nach 6 Mon. |
| Meßstelle links | 35,52 | 26,3 | 18,62 | 19,28 |
| Meßstelle rechts | 31,52 | 12,72 | 15,27 | 16,08 |
| | | | | |

| Treber-Kapseln | vorher | nach 2 Mon. | nach 4 Mon. | nach 6 Mon. |
|---|---|---|---|---|
| Meßstelle links | 30,27 | 12,45 | 13,85 | 9,52 |
| Meßstelle rechts | 29,65 | 12,72 | 14,72 | 6,55 |
| Mittelwerte (n = 4) | | | | |

Der Vergleich der NKO- mit der Treber-Kapsel-Gruppe zeigt, daß der zeitliche Ablauf der Reduktion des transepidermalen Wasserverlustes mit der Treber-Therapie viel rascher abläuft als bei der NKO-Behandlung, und zwar bereits innerhalb von zwei Monaten. Nach vier Monaten wurden fast die gleichen Werte gefunden, und nach sechs Monaten kam es dann nochmals zu einem weiteren Rückgang der Werte.

### B NMR-Spektroskopische Untersuchungen

Die Messungen wurden an einem FT-/NMR-Spektrometer (Bruker AM 400 WB) ausgeführt. Die ¹H-NMR-Messungen wurden an einem 5-mm-¹H-Selektiv-Probenkopf, die ¹³C-NMR-Messungen an einem Breitbandkopf vorgenommen. Mit Hilfe der ¹H- und ¹³C-NMR-Spektroskopie lassen sich folgende Metaboliten im Serum beobachten:
1. Lipoproteine (high density lipoproteins (HDL), low density lipoproteins (LDL) und very low density lipoproteins (VLDL))
2. Verschiedene Aminosäuren (Valin, Alanin, Glycin)
3. Lactat, Aceton, Acetat
4. N-Acetyl-Gruppen von Glycoproteinen
5. Glucose
6. 3-Hydroxybuttersäure
7. Betain, Carnitin und Cholin
8. Zitronensäure
9. Kreatin, Kreatinin
10. Gesättigte und ungesättigte Fettsäuren

Folgende Metabolite werden im Urin beobachtet:
1. Verschiedene Aminosäuren (Histidin, Valin, Alanin, Glycin)
2. Betain, Carnitin, Cholin, Acetylcarnitin
3. Zitronensäure
4. Acetat, Acetoacetat, Aceton
5. Kreatin, Kreatinin
6. Glutarsäure
7. 3-Hydroxybuttersäure
8. Lactat
9. Sarcosin
10. Succinat
11. Trimethylaminoxid
12. Mannit
13. Hippurat
14. Indoxysulfat
15. Harnstoff

Die Konzentrationsänderungen dieser Komponenten liefern Informationen über vorliegende Stoffwechselstörungen bei bestimmten Erkrankungen.
Die Analyse der ¹H- und ¹³C-NMR-Spektren im Serum und Urin der Patienten mit Dermatitis atopica zeigen Änderungen in der Konzentration der obengenannten Metabolite, die auf das Vorliegen unterschiedlich starker Stoffwechselstörungen hinweisen. Im Serum von Patienten mit Dermatitis atopica werden erhöhte LDL- und VLDL-Konzentrationen (Lipoproteine) im ¹H-NMR-Spektrum festgestellt. Nach Behandlung mit erfindungsgemaßen oral zu verabreichenden Biertreberextrakten kommt es zu einer Verringerung der LDL-und VLDL-Konzentration, wie sie bei gesunden Menschen anzutreffen ist.

Die ¹H-NMR-Spektren von Urin aus Neurodermitis Patienten zeigen vor Behandlung mit Biertreberextrakten hohe Konzentrationen von 3-Hydroxybuttersäure und Betain. Nach Behandlung zeigen sich deutlich reduzierte Konzentrationen dieser Abbauprodukte. Im Serum von Patienten mit Dermatitis atopica werden niedrige Konzentrationen von ungesättigten Fettsäuren gegenüber gesättigten Fettsäuren festgestellt. Bei normalen Seren sind die Konzentration von ungesättigten und die von gesättigten Fettsäuren ungefähr gleich. ¹³C-NMR-Spektren von Seren aus Patienten mit Dermatitis atopica zeigen nach Behandlung mit den erfindungsgemäßen Biertreberextrakten eine eindeutige Erhöhung der Konzentration an ungesättigten Fettsäuren und damit eine Verbesserung des Verhältnisses ungesättigte zu gesättigten Fettsäuren im Serum.

## Patentansprüche

1. Verwendung von Biertreberextrakt zur Herstellung von oral zu verabreichenden medizinischen Zubereitungen zur Behandlung von Hautkrankheiten, welche insbesondere mit Juckreiz und/oder entzündlichen Erscheinungen einhergehen.

2. Verwendung von Biertreberextrakt nach Anspruch 1, dadurch gekennzeichnet, daß er zur Behandlung von Dermatitis atopica, Ichthyosis, Exsikkationsekzematoid, Pruritus, Sebostase, Dermatomykosen und Quallenverbrennungen eingesetzt wird.

3. Verwendung von Biertreberextrakt nach Anspruch 1 oder 2, dadurch gekennzeichnet, daß der Biertreberextrakt mittels flüssigem oder überkritischem CO₂ oder N₂O gewonnen wurde.

4. Verwendung von Biertreberextrakt nach einem oder mehreren der Ansprüche 1 bis 3, dadurch gekennzeichnet, daß die Verabreichung in Hartgelatine-, Weichgelatine- oder Mikrokapseln erfolgt.

5. Verwendung von Biertreberextrakt nach einem oder mehreren der Ansprüche 1 bis 4, dadurch gekennzeichnet, daß die Konzentration an ungesättigten Fettsäuren in der Haut und im Serum von Menschen normalisiert oder erhöht wird.

6. Verwendung von Biertreberextrakt zur Herstellung von diätetischen Lebensmitteln.

7. Verwendung von Biertreberextrakt nach Anspruch 6, dadurch gekennzeichnet, daß die Konzentration an ungesättigten Fettsäuren in der Haut und im Serum des Menschen normalisiert oder erhöht wird.

8. Verwendung von Biertreberextrakt nach Anspruch 6 oder 7, dadurch gekennzeichnet, daß der Biertreberextrakt mittels flüssigem oder überkritischem CO₂ oder N₂O gewonnen wurde.

9. Verwendung von Biertreberextrakt nach einem oder mehreren der Ansprüche 6 bis 8, dadurch gekennzeichnet, daß die Verabreichung in Hartgelatine-, Weichgelatine- oder Mikrokapseln erfolgt.

## Claims

1. Use of spent-grain extract for the preparation of medicinal formulations to be administered orally for the treatment of skin diseases, in particular those which are accompanied by itching and/or inflammatory symptoms.

2. Use of spent-grain extract according to claim 1, wherein it is used for the treatment of dermatitis atopica, ichthyosis, desiccation eczema, pruritus, sebostasis, dermatomycoses and jellyfish burns.

3. Use of spent-grain extract according to claim 1 or 2, wherein the spent-grain extract has been obtained by means of liquid or supercritical CO₂ or N₂O.

4. Use of spent-grain extract according to one or several of the claims 1 to 3, wherein the administration occurs in the form of hard-gelatin or soft-gelatin capsules or microcapsules.

5. Use of spent-grain extract according to one or several of the claims 1 to 4, wherein the concentration of unsaturated fatty acids is normalized or increased in skin and in serum of human beings.

6. Use of spent-grain extract for the production of dietetic foodstuffs.

7. Use of spent-grain extract according to claim 6, wherein the concentration of unsaturated fatty acids is normalized or increased in skin and in serum of human beings.

8. Use of spent-grain extract according to claim 6 or 7, wherein the spent-grain extract has been obtained by means of liquid or supercritical CO₂ or N₂O.

9. Use of spent-grain extract according to one or several of the claims 6 to 8, wherein the administration occurs in the form of hard-gelatin or soft-gelatin capsules or microcapsules.

## Revendications

1. Utilisation d'extrait de drêche pour la fabrication de préparations médicales à administrer par voie orale pour le traitement de maladies de peau, en particulier celles accompagnées de démangeaisons et/ou de phénomènes inflammatoires.

2. Utilisation d'extrait de drêche selon la revendication 1, caractérisée par le fait qu'il soit utilisé pour le traitement de dermite atopique, d'ichtyose, d'eczématide, de prurit, de sébostase, de dermatomycoses et de brûlures de méduses.

3. Utilisation d'extrait de drêche selon la revendication 1 ou 2, caractérisée en ce que l'extrait de drêche soit obtenu à l'aide de CO₂ ou de N₂O liquide ou supercritique.

4. Utilisation d'extrait de drêche selon une ou plusieurs des revendications 1 à 3, caractérisée en ce que l'administration ait lieu sous forme de capsules de gélatine dure ou molle ou de microcapsules.

5. Utilisation d'extrait de drêche selon une ou plusieurs des revendications 1 à 4, caractérisée en ce que la concentration en acides gras non saturés soit normalisée ou accrue dans la peau et le sérum de l'homme.

6. Utilisation d'extrait de drêche pour la fabrication d'aliments diététiques.

7. Utilisation d'extrait de drêche selon la revendication 6, caractérisée en ce que la concentration en acides gras non saturés soit normalisée ou accrue dans la peau et le sérum de l'homme.

8. Utilisation d'extrait de drêche selon les revendications 6 ou 7, caractérisée en ce que l'extrait de drêche soit obtenu à l'aide de CO₂ ou de N₂O liquide ou supercritique.

9. Utilisation d'extrait de drêche selon une ou plusieurs des revendications 6 à 8, caractérisée en ce que l'administration ait lieu sous la forme de capsules de gélatine dure ou molle ou de microcapsules.
